# EUROPEAN PATENT SPECIFICATION

(11) **EP 0 609 963 B1**
(45) Date of publication and mention of the grant of the patent: **06.03.2002**
(21) Application number: 94200665.1
(22) Date of filing: 27.11.1989
(51) Int. Cl.: A61K 31/415, A61K 31/41, C07D 521/00

(54) **Topical compositions comprising benzimidazoles and benzotriazoles**
Topische Zubereitungen enthaltend Benzimidazol-Derivate und Benzotriazol-Derivate
Compositions topiques contenant des dérivés du benzimidazole et du benzotriazole

(30) Priority: 29.11.1988 US 277152
(43) Date of publication of application: 10.08.1994
(62) Divisional of application: 89203001.6
(73) Proprietor: JANSSEN PHARMACEUTICA N.V., 2340 Beerse (BE)
(72) Inventor: Van Wauwe, Jean Pierre Frans, B-2340 Beerse (BE); Raeymaekers, Alfons Herman Margaretha, B-2340 Beerse (BE)
(74) Representative: Quaghebeur, Luc

(56) References cited:
- EP-A- 0 260 744
- EP-A- 0 293 978
- PROGRESS IN CLINICAL AND BIOCHEMICAL RESEARCH: THERAPEUTIC PROGRESS IN UROLOGICAL CANCERS 1989 pages 205 - 209 MAHLER, C. ET AL 'THE EFFECTS OF A NEW IMIDAZOLE DERIVATIVE IN ADVANCED PROSTATIC CANCER. A PRELIMINARY REPORT'
- THE JOURNAL OF MEDICINAL CHEMISTRY vol. 32, no. 10 , October 1989 pages 2231 - 2239 VAN WAUWE, J.P. ET AL 'IS THERE A CASE FOR P-450 INHIBITORS IN CANCER TREATMENT?'
- PHARMACOLOGY AND EXPERIMENTAL THERAPEUTICS vol. 245, no. 2 , April 1988 pages 718 - 722 VAN WAUWE, J.P. ET AL 'KETOCONAZOLE INHIBITS THE IN VITRO AND IN VIVO METABOLISM OF ALL-TRANS-RETINOIC ACID'

## Description

Retinoids, in particular retinoic acid and its derivatives, are substances which are known to have a broad spectrum of biological activity. More specifically, these substances affect the differentiation, maintenance and proliferation of various cell types. The ability of retinoids, such as, all-*trans*-retinoic acid, 13-*cis*-retinoic acid, and their derivatives to modulate differentiation in several cell types, whether they are of epithelial or mesenchymal origin, is extensively studied and reviewed in J. Clin. Chem. Clin. Biochem., 26, 479-488 (1983); Pharmacological Reviews, 36, 935-1005 (1984) and Arch. Dermatol., 117, 160-180 (1981).

It is known that certain retinoids, particularly the retinoic acids, are used topically for treatment of acne as set forth in U.S. Patent No. 3,729,568. Other known uses of retinoic acid were reviewed in the Journal of American Academy of Dermatology, 4, 505-516 (1981) and the Journal of Medical Chemistry, 25, 1269-1277 (1982) and include, in addition to acne treatment, treatment of senile comedones, nevus comedonicus, linear verrucous nevus, plantar warts, pseudofolliculitis, keratoacanthoma, solar keratosis of extremities, callosities, keratosis palmaris et plantaris, Darier's disease, ichthyosis, psoriasis, acanthosis nigricans, lichen planus, molluscum contagiosum, reactive perforating collagenosis, melasma, corneal epithelial abrasion, Fox-Fordyce disease, cutaneous metastatic melanoma and keloids or hypertrophic scars.

Retinoids such as, all-*trans*-retinoic acid, 13-*cis*-retinoic acid and their derivatives, have also been used in the treatment of carcinomas.

EP-0,293,978, published 7 December 1988, and the Journal of Medicinal Chemistry vol. 32, no. 10, pages 2231 - 2239, published in October 1989, disclose (1H-azol-1-ylmethyl) substitued benzotriazole derivatives having potential use in the treatment of estrogen dependent disorders such as cancer.

Pharmacology and Experimental Therapeutics vol 245, no. 2, pages 718 - 722, published in April 1988 describes the effect of ketoconazole on the metabolism of all-trans retinoic acid and forecasts the feasibility to develop specific drugs that are capable of delaying the biodegradation and prolonging the biological half-life or retinoic acid.

There are however a number of drawbacks associated with the therapeutic applications of retinoids. The topical applications of retinoids on the one hand often cause significant irritation and peeling due to the relatively high concentrations of retinoid which have to be applied. Systemic applications on the other hand are limited by the toxicity and rapid degradation of the administered retinoids.

The compositions of the invention overcome the problems associated with art known retinoid therapy by suppressing the metabolism of endogenously or exogenously administered retinoic acid.

The present invention concerns pharmaceutical or cosmetical composition suitable for topical application comprising an inert carrier, an effective amount of a retinoic acid or a derivative thereof, or a stereochemically isomeric form thereof and an effective amount of a compound of formula 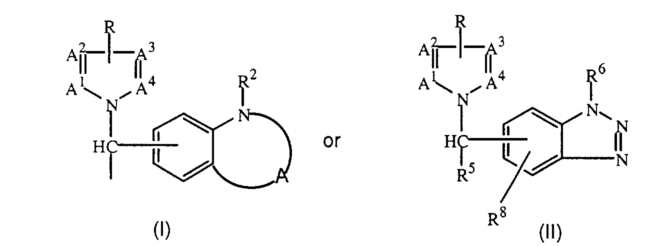 an acid addition salt thereof or a stereochemically isomeric form thereof, wherein in the compound of formula (I) the 1H-azol-1-ylmethyl moiety is substituted on either the 5 or 6 position of the benzimidazole ring and wherein R, R¹, R², -A¹=A²-A³=A⁴-and A in formula (I) have the following meaning
-A¹=A²-A³=A⁴- is a bivalent radical having the formula

   -CH=N-CH=CH- (x);

   -CH=N-CH=N- (y);

   or

   -CH=N-N=CH- (z);
R is hydrogen;
R1 is hydrogen; C1-6alkyl; C3-7cycloalkyl; phenyl; substituted phenyl; thienyl or furanyl optionally substituted with halo;
R² is hydrogen; C₃₋₇cycloalkyl; phenyl; substituted phenyl; pyridinyl; C₁₋₆alkyl optionally monosubstituted with phenyl, C₃₋₇cycloalkyl, pyridinyl or thienyl; hydroxyl, C₁₋₆alkyloxy optionally monosubstituted with phenyl, pyridinyl, thienyl or C₃₋₆cycloalkyl; C₃₋₆alkenyloxy optionally monosubstituted with phenyl; or C₃₋₆alkynyloxy;
A is a bivalent radical having the formula

   -CR³=N- (a)

   or 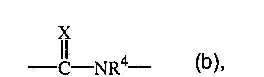 wherein the carbon atom in the bivalent radical (a) and (b) is connected to -NR²;
R³ is hydrogen; C₁₋₄alkyl substituted with up to 4 halo atoms; C₃₋₇cycloalkyl; phenyl; substituted phenyl; imidazolyl; thiazolyl; thienyl; furanyl; quinolinyl; pyridinyl optionally substituted with amino, C₁₋₁₀alkyl; C₁₋₅alkyl optionally monosubstituted with phenyl, pyridinyl, imidazolyl, thienyl, indolyl or hydroxyl; C₁₋₄alkyloxy optionally monosubstituted with phenyl; C₂₋₆alkenyl optionally monosubstituted with pyridinyl, furanyl, imidazolyl or phenyl; carboxyl; C₁₋₄alkyloxycarbonyl; phenylcarbonyl; or hydroxy and phenylmethyl;
said X being O or S;
R⁴ is hydrogen or phenylC₁₋₄alkyl; wherein substituted phenyl is phenyl substituted with 1, 2 or 3 substituents each independently selected from halo, hydroxy, trifluoromethyl, C₁₋₆alkyl, C₁₋₆alkyloxy, cyano, amino, mono- and di(C₁₋₆alkyl)amino, nitro, carboxyl, formyl and C₁₋₆alkyloxycarbonyl; and wherein in the compound of formula (II) the 1H-azol-1-ylmethyl moiety is substituted on either the 5 or 6 position of the benzotriazole ring, and wherein
R, R⁵, R⁶, R⁸ and -A¹=A²-A³=A⁴- in formula (II) have the following meaning
-A¹=A²-A³=A⁴- is a bivalent radical having the formula

   -CH=N-CH=CH- (x);

   -CH=N-CH=N- (y);

   or

   -CH=N-N=CH- (z);
R is hydrogen;
R⁵ is hydrogen; C₁₋₆alkyl; phenyl; substituted phenyl; C₃₋₇cycloalkyl; thienyl or furanyl optionally substituted with halo;
R⁶ is hydrogen; C₁₋₆alkyl; C₃₋₆alkenyl; C₃₋₆alkynyl; C₃₋₇cycloalkyl; phenyl; substituted phenyl; bicyclo[2.2.1]heptan-2-yl; 2,3-dihydro-1H-indenyl; 1,2,3,4-tetrahydronaphthalenyl; C₁₋₆alkyl monosubstituted with phenyl, substituted phenyl, napthalenyl, thienyl, furanyl, C₁₋₄alkylfuranyl, C₃₋₇cycloalkyl, hydroxy, C₁₋₄alkyloxy or R⁶ is a radical -OR⁷
R⁷ being hydrogen , C₁₋₆alkyl, C₃₋₆alkenyl, phenylC₃₋₆alkenyl,C₃₋₆alkynyl, pyrimidinyl, diphenylmethyl, (1-C₁₋₄alkyl-4-piperidinyl), C₁₋₆alkyl substituted with halo, hydroxy, amino, mono- or di(C₁₋₆alkyl) amino, trifluoromethyl, carboxyl, C₁₋₆alkyloxycarbonyl, phenyl, substituted phenyl, thienyl, furanyl, C₁₋₄alkylfuranyl, pyridinyl, phenoxy, phenylthio, 2,3-dihydro-1,4-benzodioxinyl, 1H-benzimidazolyl, C₁₋₄alkyl substituted 1H-benzimidazolyl, (1,1 '-biphenyl)-4-yl or 2,3-dihydro-2-oxo-1H-benzimidazolyl; and
R⁸ is hydrogen; wherein substituted phenyl is phenyl substituted with up to 3 substituents each independently selected from halo, hydroxy, hydroxymethyl, trifluoromethyl, C₁₋₆alkyl, C₁₋₆alkyloxy, C₁₋₆alkyloxycarbonyl, carboxyl, formyl, (hydroxyimino)methyl, cyano, amino, mono- and di(C₁₋₆alkyl)amino and nitro. Preferably said substituted phenyl is phenyl substituted with one or two substituents each independently selected from halo, C₁₋₆alkyl, C₁₋₆alkyloxy and trifluoromethyl.

As used in the foregoing definitions the term halo is generic to fluoro, chloro, bromo and iodo; the term "C₁₋₆alkyl" is meant to include straight chained and branched saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl and the like; "C₁₋₁₀alkyl" is meant to include C₁₋₆alkyl radicals, as defined hereinabove, and the higher homologs thereof having from 7 to 10 carbon atoms; the term "C₃₋₇cycloalkyl" is generic to cyclopropyl, cyclobutyl, cyclopentyl, cyclohexyl and cycloheptyl. "C₂₋₆alkenyl" defines straight chained and branched hydrocarbon radicals containing one double bond having from 2 to 6 carbon atoms such as, for example, ethenyl, 2-propenyl, 3-butenyl, 2-butenyl, 2-pentenyl, 3-pentenyl, 3-methyl-2-butenyl and the like; "C₂₋₆alkynyl" defines straight chained and branched hydrocarbon radicals containing one triple bond and having from 2 to 6 carbon atoms such as, for example, 2-propynyl, 2-butynyl, 3-butynyl, 2-pentynyl, 3-pentynyl, 4-pentynyl and the like; and when a C₂₋₆alkenyl or a C₂₋₆alkynyl is substituted on a heteroatom, then the carbon atom of said C₂₋₆alkenyl or said C₂₋₆alkynyl connected to said heteroatom preferably is saturated.

It is to be understood that the 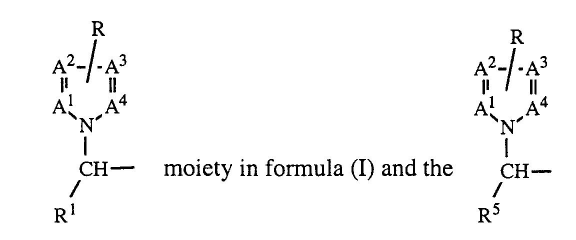 moiety in formula (II), both hereinafter referred as the 1H-azol-1-ylmethyl moiety, may be substituted on the 5 or 6 position, with the 5 position being preferred.

It is evident that the compounds of formula (I) may also contain in their structure a tautomeric system and consequently these compounds can be present in each of their tautomeric forms.

More particular compounds are those particular compounds of formula (I) wherein R¹ is hydrogen, C₁₋₆alkyl, phenyl, substituted phenyl, thienyl or furanyl; R² is hydrogen, C₁₋₆alkyl, or C₁₋₄alkyl substituted with phenyl; R³ is hydrogen, C₁₋₆alkyl, phenyl, pyridinyl, C₁₋₆alkyl, C₁₋₆alkyl monosubstituted with phenyl or C₂₋₆alkenyl optionally monosubstituted with furanyl or phenyl; and R⁴ is hydrogen.

Other more particular compounds of the present invention are those particular compounds of formula (II) wherein R⁵ is hydrogen, C₁₋₆alkyl, phenyl, substituted phenyl, thienyl or furanyl; R⁶ is hydrogen, C₁₋₆alkyl, C₁₋₆alkyl substituted with phenyl, or a radical of formula -OR⁷ with R⁷ being hydrogen or C₁₋₆alkyl.

Preferred compounds are those particular compounds of formula (I) wherein R¹ is C₁₋₄alkyl, phenyl, phenyl substituted with one or two halo, C₁₋₄alkyl or C₁₋₄alkyloxy substituents, or thienyl; R² is hydrogen or C₁₋₄alkyl; and R³ is hydrogen or C₁₋₄alkyl.

Other preferred compounds are those particular compounds of fomula (II) wherein R⁵ is C₁₋₄alkyl, phenyl or phenyl substituted with one or two halo, C₁₋₄alkyl or C₁₋₄alkyloxy substituents; and R⁶ is hydrogen or C₁₋₄alkyl.

More preferred compounds are those preferred compounds of formula (I) wherein R¹ is phenyl or halophenyl, and R² and R³ are both independently hydrogen or C₁₋₄alkyl.

Other more preferred compound are those preferred compounds of formula (II) wherein R⁵ is phenyl or halophenyl and R⁶ is hydrogen or C₁₋₄alkyl.

Most preferred compounds are 5-[(1H-imidazol-1-yl)phenylmethyl]-1H-benzimidazole, (±)-5-[(1H-imidazol-1-yl)phenylmethyl]-2-methyl-1H-benzimidazole, 5-[(1H-imidazol-1-yl)phenylmethyl]-1-methyl-1H-benzimidazole, 5-[1-(1H-imidazole-1-yl)-2-methylpropyl]-2-methyl-1H-benzimidazole, 5-[(3-chlorophenyl) (1H-imidazol-1-yl)methyl]-1H-benzimidazole or (±)-5-[(1H-imidazol-1-yl)phenylmethyl]-2-methyl-1H-benzimidazole, the pharmaceutically acceptable acid addition salts and possible stereoisomers thereof.

From formulae (I) and (II) it is evident that the compounds of this invention may have several asymmetric carbon atoms in their structure. Each of these chiral centers may be present in a R- and a S-configuration, this R- and S-notation being in correspondence with the rules described by R.S. Cahn, C. Ingold and V. Prelog in Angew. Chem., Int. Ed. Engl., 5, 385, 511(1966).

Pure stereochemically isomeric forms of the compounds of formulae (I) and (II) may be obtained by the application of art-known procedures. Diastereoisomers may be separated by physical separation methods such as selective crystallization and chromatographic techniques, e.g., counter current distribution, and enantiomers may be separated from each other by the selective crystallization of their diastereomeric salts with optically active acids.
Pure stereochemically isomeric forms may also be derived from the corresponding pure stereochemically isomeric forms of the appropriate starting materials, provided that the reaction occurs stereospecifically.
Stereochemically isomeric forms of the compounds of formulae (I) and (II) are naturally intended to be embraced within the scope of the invention.

Some of the compounds of formula (I) and (II) which can be used as active ingredient in the compositions according to the present invention are listed in the following tables with the purpose of illustrating the invention and not to limit it thereto.

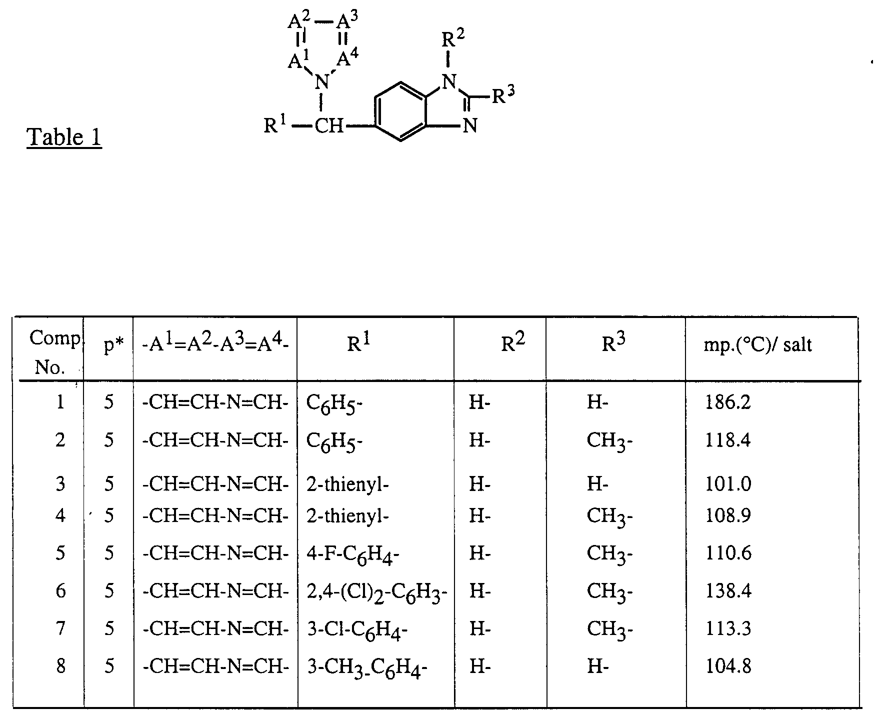

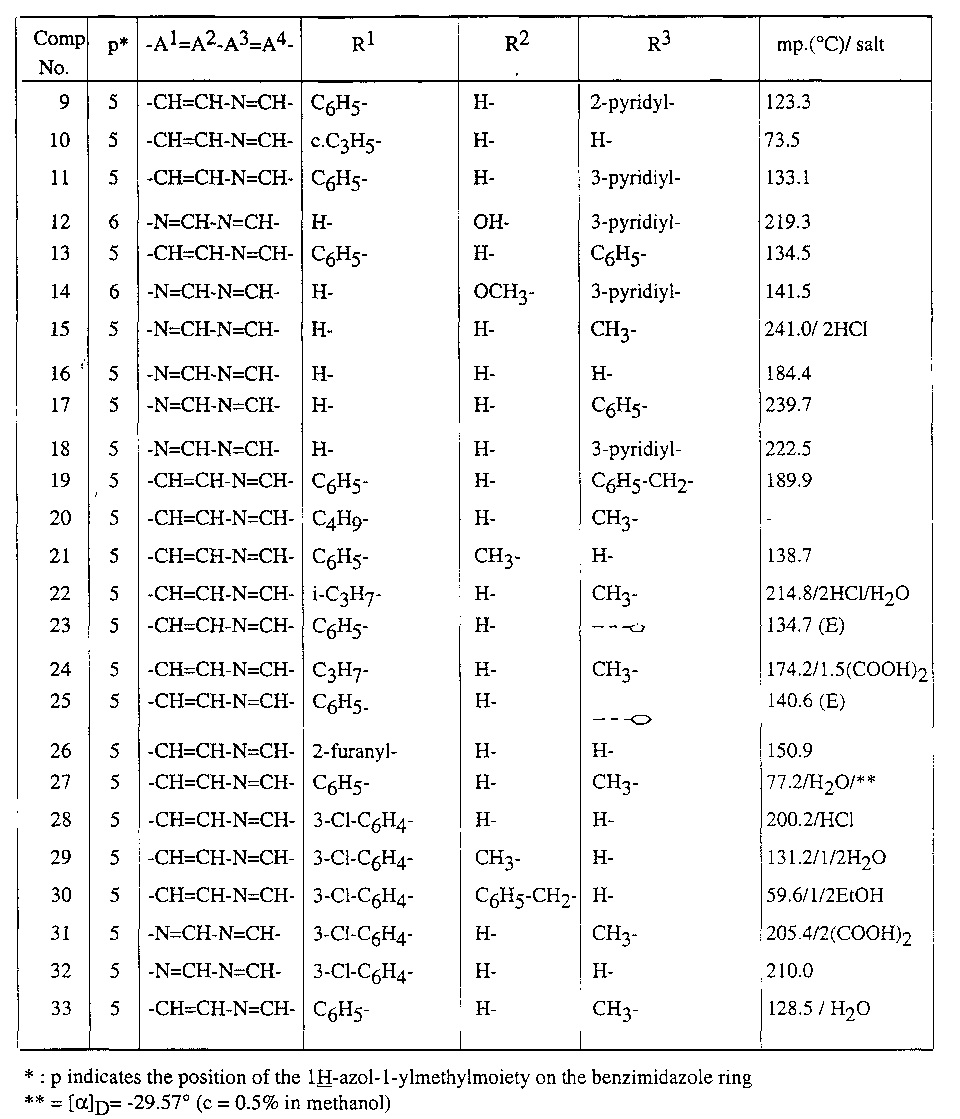

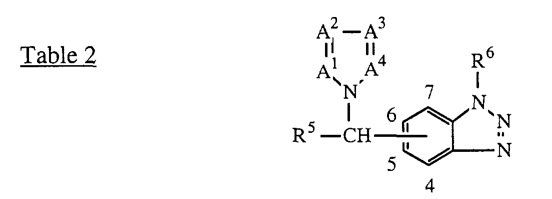

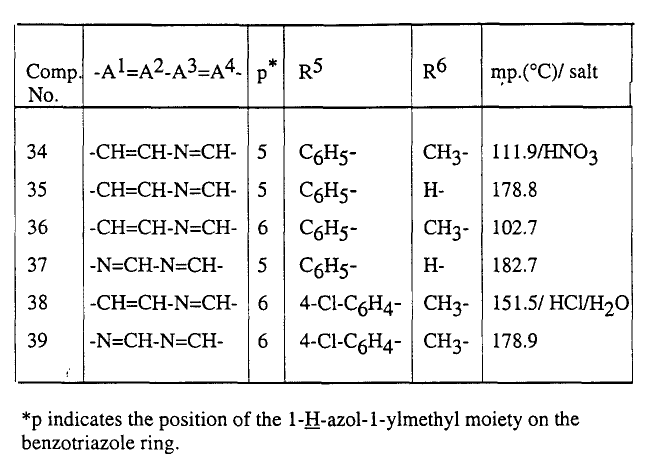

The use of the compounds of formula (I) and (II), their pharmaceutically acceptable acid addition salts and their possible stereochemically isomeric forms for the manufacture of a medicament for treating mammals suffering from disorders which are characterized by an increased proliferation and/or abnormal differentiation of epithelial cells the growth of which is sensitive to the action of retinoids, is based on their useful property to delay the metabolism of retinoids, such as, all-*trans*-retinoic acid, *13*-*cis*-retinoic acid and their derivatives. The latter results in more sustained / higher tissue concentrations of retinoids and improved control of differentiation and growth of various cell types. Said property to delay the metabolism of retinoids can easily be evidenced in various in *vivo* experiments. A particular test procedure is described hereinafter as the "Metabolism of endogenous or exogenously administered all-*trans*-retinoic acid"-test . As such, the compounds of formula (I) and (II) can be used to control the rate of growth and differentiation of normal, preneoplastic and neoplastic epithelial cells. The ability of retinoids, such as, 13-*cis*-retinoic acid, all-*trans*-retinoic acid and their derivatives to modulate differentiation and proliferation in several cell types is extensively studied and reviewed in J. Clin. Chem. Clin, Biochem., 26, 479-488 (1983); Pharmacological Reviews 36, 935-1005, (1984), Arch. Dermatol. 117, 160-180; (1981) and Journal of Medicinal Chemistry 25, 1269-1277, (1982).

The compounds of formulae (I) and (II), their pharmaceutically acceptable acid addition salts and their possible stereochemically isomeric forms are therefore useful to treat disorders which are characterized by an increased proliferation and/or abnormal differentiation of epithelial cells. Other uses include the ability to cure and/or reduce a variety of disorders of keratinization such as, for example, acne, psoriasis, lamellar ichthyosis, plantar warts, callosites, acanthosis nigricans, lechen planus, molluscum, melasma, corneal epithelial abrasion, geograpic tongue, Fox-Fordyce disease, cutaneous metastatic melanoma and heloids, epidermolytic hyperkeratosis, Darier's disease, pityriasis rubra pilaris, congenital ichthyosiform erythroderma, hyperkeratosis palmaris et plantaris, and similar disordes.

Those of skill in treating disorders which are characterized by an excessive proliferation and/or abnormal differentiation of tissues could determine the effective amount from the test results presented hereinafter. In general it is contemplated than an effective amount would be from 0.001 mg/kg to 50 mg/kg body weight and more preferably from 0.01 mg/kg to 10 mg/kg body weight.

The compounds of formulae (I) and (II) are most preferably applied in the form of appropriate compositions. As appropriate compositions there may be cited all compositions usually employed for topically administering drugs. To prepare the pharmaceutical compositions of this invention, an effective amount of the particular compound, optionally in acid-addition salt form, as the active ingredient is combined in intimate admixture with a pharmaceutically acceptable carrier, which carrier may take a wide variety of forms depending on the form of preparation desired for administration.

As appropriate compositions for topical application there may be cited all compositions usually employed for topically administering drugs, e.g., creams, gellies, dressings, shampoos, tinctures, pastes, ointments, salves, powders, liquid or semi-liquid formulation and the like. Application of said compositions may be by aerosol e.g. with a propellent such as nitrogen carbon dioxide, a freon, or without a propellent such as a pump spray, drops, lotions, or a semisolid such as a thickened composition which can be applied by a swab. In particular compositions, semisold compositions such as salves, creams, pastes, gellies, ointments and the like will conveniently be used.

Other such compositions are preparations of the cosmetic type, such as toilet waters, packs, lotions, skin milks or milky lotions. Said preparations contain, besides the active ingredient of formula (I) or (II), components usually employed in such preparations. Examples of such components are oils, fats, waxes, surfactants, humectants, thickening agents, antioxidants, viscosity stabilizers, chelating agents, buffers, preservatives, perfumes, dyestuffs, lower alkanols, and the like. If desired, further ingredients may be incorporated in the compositions, e.g. antiinflammatory agents, antibacterials, antifungals, disinfectants, vitamins, sunscreens, antibiotics, or other anti-acne agents.

Examples of oils comprise fats and oils such as olive oil and hydrogenated oils; waxes such as beeswax and lanolin; hydrocarbons such as liquid paraffin, ceresin, and squalane; fatty acids such as stearic acid and oleic acid; alcohols such as cetyl alcohol, stearyl alcohol, lanolin alcohol, and hexadecanol; and esters such as isopropyl myristate, isopropyl palmitate and butyl stearate. As examples of surfactants there may be cited anionic surfactants such as sodium stearate, sodium cetylsulfate, polyoxyethylene lauryl-ether phosphate, sodium N-acyl glutamate; cationic surfactants such as stearyldimethyl-benzylammonium chloride and stearyltrimethylammonium chloride; ampholytic surfac-tants such as alkylaminoethylglycine hydrochloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, polyoxyethylene polyoxypropylene glycol (e.g. the materials sold under the trademark "Pluronic"), polyoxyethylene castor oil, and polyoxyethylene lanolin. Examples of humectants include glycerin, 1,3-butylene glycol, and propylene glycol; examples of lower alcohols include ethanol and isopropanol; examples of thickening agents include xanthan gum, hydroxypropyl cellulose, hydroxypropyl methyl cellulose, polyethylene glycol and sodium carboxymethyl cellulose; examples of antioxidants comprise butylated hydroxytoluene, butylated hydroxyanisole, propyl gallate, citric acid and ethoxyquin; examples of chelating agents include disodium edetate and ethanehydroxy diphosphate; examples of buffers comprise citric acid, sodium citrate, boric acid, borax, and disodium hydrogen phosphate; and examples of preservatives are methyl parahydroxybenzoate, ethyl parahydroxybenzoate, dehydroacetic acid, salicylic acid and benzoic acid.
For preparing ointments, creams, toilet waters, skin milks, and the like, typically from 0.01 to 10% in particular from 0.1 to 5% and more in particular from 0.2 to 2.5% of the active ingredient of formula (I) or (II) will be incorporated in said compositions. In ointments or creams, the carrier for example consists of 1 to 20%, in particular 5 to 15% of a humectant, 0.1 to 10% in particular from 0.5 to 5% of a thickener and water; or said carrier may consist of 70 to 99%, in particular 20 to 95% of a surfactant, and 0 to 20%, in particular 2.5 to 15% of a fat; or 80 to 99.9% in particular 90 to 99% of a thickener; or 5 to 15% of a surfactant, 2-15% of a humectant, 0 to 80% of an oil, very small (<2%) amounts of preservative, colouring agent and/or perfume, and water. In a toilet water, the carrier for example consists of 2 to 10% of a lower alcohol, 0.1 to 10% or in particular 0.5 to 1% of a surfactant, 1 to 20%, in particular 3 to 7% of a humectant, 0 to 5% of a buffer, water and small amounts (<2%) of preservative, dyestuff and/or perfume. In a skin milk, the carrier typically consists of 10-50% of oil, 1 to 10% of surfactant, 50-80% of water and 0 to 3% of preservative and/or perfume. In the afore-mentioned preparations, all % symbols refer to weight by weight percentage. The humectant, surfactant, oil, etc... referred to in said preparations may be any such component used in the cosmetic arts but preferably will be one or more of the components mentioned hereinabove. Further, when in the above compositions one or more of the components make up the major part of the composition, the other ingredients can evidently be not present at their indicated maximum concentration and therefore will make up the remainder of the composition.

Particular compositions for use in the present invention are those wherein the active ingredient of formula (I) or (II) is formulated in liposome-containing compositions. Liposomes are artificial vesicles formed by amphiphatic molecules such as polar lipids, for example, phosphatidyl cholines, ethanolamines and serines, sphingomyelins, cardiolipins, plasmalogens, phosphatidic acids and cerebiosides. Liposomes are formed when suitable amphiphathic molecules are allowed to swell in water or aqueous solutions to form liquid crystals usually of multilayer structure comprised of many bilayers separated from each other by aqueous material (also referred to as coarse liposomes). Another type of liposome known to be consisting of a single bilayer encapsulating aqueous material is referred to as a unilamellar vesicle. If water-soluble materials are included in the aqueous phase during the swelling of the lipids they become entrapped in the aqueous layer between the lipid bilayers.

Water-soluble active ingredients such as, for example, most of the salt forms of the compound of formula (I) or (II) are encapsulated in the aqueous spaces between the molecular layers. The lipid soluble active ingredient of formula (I) or (II) is predominantly incorporated into the lipid layers, although polar head groups may protrude from the layer into the aqueous space. The encapsulation of these compounds can be achieved by a number of methods. The method most commonly used involves casting a thin film of phospholipid onto the walls of a flask by evaporation from an organic solvent. When this film is dispersed in a suitable aqueous medium, multilamellar liposomes are formed. Upon suitable sonication, the coarse liposomes form smaller similarly closed vesicles.

Water-soluble active ingredients are usually incorporated by dispersing the cast film with an aqueous solution of the compound. The unencapsulated compound is then removed by centrifugation, chromatography, dialysis or other art-known suitable procedures. The lipid-soluble active ingredient is usually incorporated by dissolving it in the organic solvent with the phospholipid prior to casting the film. If the solubility of the material in the lipid phase is not exceeded or the amount present is not in excess of that which can be bound to the lipid, liposomes prepared by the above method usually contain most of the material bound in the lipid bilayers; separation of the liposomes from unencapsulated material is not required.

A particularly convenient method for preparing liposome formulated forms of the active ingredient of formula (I) or (II) is the method described in EP-A-253,619, incorporated herein by reference. In this method, single bilayered liposomes containing encapsulated active ingredients are prepared by dissolving the lipid component in an organic medium, injecting the organic solution of the lipid component under pressure into an aqueous component while simultaneously mixing the organic and aqueous components with a high speed homogenizer or mixing means, whereupon the liposomes are formed spontaneously.

The single bilayered liposomes containing the encapsulated active ingredient of formula (I) or (II) can be employed directly or they can be employed in a suitable pharmaceutically acceptable carrier for topical administration. The viscosity of the liposomes can be increased by the addition of one or more suitable thickening agents such as, for example xanthan gum, hydroxypropyl cellulose, hydroxypropyl methylcellulose and mixtures thereof. The aqueous component may consist of water alone or it may contain electrolytes, buffered systems and other ingredients, such as, for example, preservatives. Suitable electrolytes which can be employed include metal salts such as alkali metal and alkaline earth metal salts. The preferred metal salts are calcium chloride, sodium chloride and potassium chloride. The concentration of the electrolyte may vary from zero to 260 mM, preferably from 5 mM to 160 mM. The aqueous component is placed in a suitable vessel which can be adapted to effect homogenization by effecting great turbulence during the injection of the organic component. Homogenization of the two components can be accomplished within the vessel, or, alternatively, the aqueous and organic components may be injected separately into a mixing means which is located outside the vessel. In the latter case, the liposomes are formed in the mixing means and then transferred to another vessel for collection purpose.
The organic component consists of a suitable non-toxic, pharmaceutically acceptable solvent such as, for example ethanol, glycerol, propylene glycol and polyethylene glycol, and a suitable phospholipid which is soluble in the solvent. Suitable phospholipids which can be employed include lecithin, phosphatidylcholine, phosphatidylethanol-amine, phosphatydylserine; phosphatidylinositol, lysophosphatidylcholine and phospha-tidyl glycerol, for example. Other lipophilic additives may be employed in order to selectively modify the characteristics of the liposomes. Examples of such other additives include stearylamine, phosphatidic acid, tocopherol, cholesterol and lanolin extracts.
It may be advantageous to use micronized forms of the active ingredient of formula (I) or (II), i.e., material having an average particle size of less than 10 microns, as the high surface area will facilitate the dissolution of the liposomal components.
In addition, other ingredients which can prevent oxidation of the phospholipids may be added to the organic component. Examples of such other ingredients include tocopherol, butylated hydroxyanisole, butylated hydroxytoluene, ascorbyl palmitate and ascorbyl oleate. Preservatives such as benzoic acid, methyl paraben and propyl paraben may also be added.
The liposome-formulated forms of the active ingredient of formula (I) or (II), particularly those obtained in the above-referred method of preparing such liposome formulated forms, may be used as such or in combination with any of the afore-mentioned carriers to prepare ointments, creams, gelées, toilet waters, etc...
Apart from the above-described compositions, use may be made of covers, e.g. plasters, bandages, dressings, gauze pads and the like, containing an appropriate amount of a composition as referred hereinabove. In some cases use may be made of plasters, bandages, dressings, gauze pads and the like which have been impregnated or sprinkled with a liquid formulation containing the active agent, e.g. with an aseptic aqueous solution, or strewn with a powdery solid composition, or smeared, covered or coated with a semi-liquid composition.

It can be demonstrated that the combined effect of the retinoic acids and the compounds of formula (I) or (II) is greater than the sum of their respective effects when administered separately. As evidenced by the data obtained in the "vaginal keratinization"-test described hereinafter. The above described retinoic acid containing compositions are particularly useful for treating acne or for retarding the effects of aging of the skin and generally improve the quality of the skin, particularly human facial skin. A pharmaceutical or cosmetical composition containing retinoic acid or a derivative thereof as the active ingredient in intimate admixture with a dermatologically acceptable carrier can be prepared according to conventional compounding techniques, such as those known for topical application of retinoic acid and its derivatives. Conventional pharmaceutical compounding techniques for topical application of retinoic acid are described for example in, U.S. Pat. Nos. 3,906,108 and 4,247,547, which are incorporated herein by reference. Preferred composition for topical application are in form of a cream, ointment or lotion comprising from 0.005 to 0.5% (particularly from 0.01 to 0.1%) all-*trans*-retinoic acid, 13-*cis*-retinoic acid or a derivative thereof and from 0.1 to 5% of a compound of formula (I) and/or (II), a dermatologically acceptable acid addition salt thereof or a stereochemically isomeric form thereof, in a semi-solid or liquid diluent or carrier.
These preferred composition should preferably be non-irritating and as far as possible they should be odorless and non-toxic. For convenience in applying to the skin, the composition usually contain, besides water or an organic solvent, several of certain organic emollients, emulsifiers for the aqueous and/or non aqueous phases of the compositions, wetting agents preservatives and agents that facilitate the penetration and remainence of the active agents in the skin.
In use, the retinoic acid containing compositions of the invention are applied topically to the area to be treated or protected, at regular intervals, as needed, generally about 7 to about 21 times per week. The duration of the treatment will depend upon the nature and severity of the condition to be treated as well as the frequency of application of the composition.

The following examples are intended to illustrate the scope of the present invention in all its aspects, and not to limit it thereto.

### Experimental part

### A. Pharmaceutical Examples

### Example 1

### Metabolism of exogenously administered all-trans-retinoic acid

Male Wistar rats weighing 200∼210 g were orally treated with vehicle (PEG 200) or with 40 mg/kg of a compound of formula (I). One hour later, the animals were anesthetized with ether and injected intrajugularly with 0.50 ml saline solution containing 20 µg of all-*trans*-retinoic acid. Two hours after this injection, rats were killed by decapitation and blood was collected on heparin. Blood samples were centrifuged (1000 g, 15 min) and plasma was recovered to determine the quantity of plasmatic all-*trans*-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 1, 2, 3, 4, 5, 6, 7, 8, 9, 11, 13, 19, 20, 21, 22, 24, 28, 29, 30, 33 and 34 enhanced the recovery of all-*trans*-retinoic acid from the plasma to a least 8 ng/ml after dosing with 40 mg/kg.

### Example 2

### Metabolism of endogenous all-trans-retinoic acid

Male Wistar rats weighing 200∼210 g were orally treated with vehicle (PEG 200) or with 40 mg/kg of a compound of formula (I). Two hours after drug administration, the rats were killed by decapitation and blood was collected on heparin. Blood samples were centrifuged (1000 g, 15 min) and plasma was recovered to determine the quantity of plasmatic all-*trans*-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 1, 2, 7, 13, 21, 22, 27, 28 and 33 enhanced the recovery of all-*trans*-retinoic acid from the plasma to a least 1 ng/ml.

### Example 3

### Vaginal keratinization.

Ovariectomized rats were injected subcutaneously with a sesame oil solution containing 100 µg of estradiol undecylate (Progynon Dépôt®, Schering) in a volume of 0.1 ml per 100 g body weight. One and two days later, the animals were treated intravaginally with 200 µl of vehicle (PEG 200), all-*trans*-retinoic acid (1 or 4 µg) or all-*trans*-retinoic acid (1 µg) together with 3 mg of a compound of formula (I). One day after the second topical treatment, the animals were sacrificed. Vaginas were immediately dissected and trimmed of fat and connective tissue. The third middle of the organ (0.5 cm length) was fixed in liquid nitrogen for histological analysis. Hereto, a series of 10 µm cross-section were cut at -25°C, mounted onto gelatin-coated glass slides and stained with hematoxylin and eosin. The slides were examined under light microscopy at 100-400 x magnification. The condition of the vaginal mucosa was scored (keratinization score) as
0 : absence of keratinized squamae attached to the epithelial cells.
+ : presence of keratinized squamae partially covering the epithelial cells.
++ : presence of keratinized squamae covering the entire vaginal epithellium.

### Results

| | score |
|---|---|
| vehicle (PEG 200) | ++ |
| 4 µg retinoic acid | 0 |
| 1 µg retinoic acid | ++ |
| 1µg retinoic acid + 3 mg comp. No.21 | 0 |

### C) Composition Examples

The following formulations exemplify typical pharmaceutical and cosmetical compositions suitable for topical administration to warm-blooded animals in accordance with the present invention.

"Active ingredient" (A.I.) as used throughout these examples relates to a compound of formula (I), a pharmaceutically acceptable acid addition salt or a stereochemically isomeric form thereof.

### Example 4 : 2% TOPICAL GEL

To a solution of 200 mg of hydroxypropyl β-cyclodextrine in purified water is added 20 mg of active ingrediënt of formula (I) or (II) while stirring. Hydrochloric acid is added until complete solution and then sodium hydroxide is added until pH 6.0. This solution is added to a dispersion of 10 mg of carrageenan PJ in 50 mg of propylene glycol while mixing. While mixing slowly the mixture is heated to 50°C and allowed to cool to about 35°C whereupon 50 mg of ethyl alcohol 95% is added. The rest of the purified water is added q.s. ad 1 g and the mixture is mixed to homogenous.

### Example 5 : 2% TOPICAL CREAM

To a solution of 200 mg of hydroxypropyl β-cyclodextrine in purified water is added 20 mg of active ingrediënt of formula (I) or (II) while stirring. Hydrochloric acid is added until complete solution and next sodium hydroxide is added until pH 6.0. While stirring, 50 mg of glycerol and 35 mg of polysorbate 60 are added and the mixture is heated to 70°C. The resulting mixture is added to a mixture of 100 mg of mineral oil, 20 mg of stearyl alcohol, 20 mg of cetyl alcohol, 20 mg of glycerol monostearate and 15 mg of sorbate 60 having a temperature of 70°C while mixing slowly. After cooling down to below 25°C, the rest of the purified water is added q.s. ad 1 g and the mixture is mixed to homogenous.

### Example 6 : 2% LIPOSOME FORMULATION

A mixture of 2 g of active ingredient of formula (I) or (II) microfine, 20 g of phosphatidyl choline, 5 g of cholesterol and 10 g of ethyl alcohol is stirred and heated at 55-60°C until complete solution and is added to a solution of 0.2 g of methyl paraben, 0.02 g of propyl paraben, 0.15 g of disodium edetate and 0.3 g of sodium chloride in purified water while homogenizing. 1.5 g of hydroxypropylmethylcellulose in purified water is added ad 100 g and the mixing is continued until swelling is complete.

### Example 7 : 2% LIPOSOME FORMULATION

A mixture of 10 g of phosphatidyl choline and 1 g of cholesterol in 7.5 g of ethyl alcohol is stirred and heated at 40°C until complete solution. 2 g of active ingredient of formula (I) or (II) microfine is dissolved in purified water by mixing while heating at 40°C. The alcoholic solution is added slowly to the aqueous solution while homogenizing during 10 minutes. 1.5 g of hydroxypropylmethylcellulose in purified water is added while mixing until swelling is complete. The resulting solution is adjusted to pH 5.0 with sodium hydroxide 1 N and diluted with the rest of the purified water ad 100 g.

## Claims

1. A pharmaceutical or cosmetical composition suitable for topical application comprising an inert carrier, an effective amount of a retinoic acid or a derivative thereof, or a stereochemically isomeric form thereof and an effective amount of a compound of formula 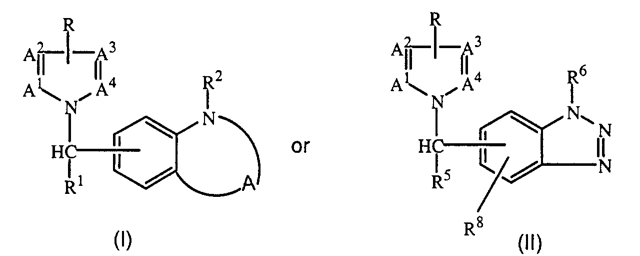 an acid addition salt thereof or a stereochemically isomeric form thereof, wherein
in the compound of formula (I) the 1H-azol-1-ylmethyl moiety is substituted on either the 5 or 6 position of the benzimidazole ring and wherein R, R¹, R², -A¹=A²-A³=A⁴- and A in formula (I) have the following meaning
-A¹=A²-A³=A⁴- is a bivalent radical having the formula
-CH=N-CH=CH- (x);
-CH=N-CH=N- (y);
or
-CH=N-N=CH- (z);
R is hydrogen;
R¹ is hydrogen; C₁₋₆alkyl; C₃₋₇cycloalkyl; phenyl; substituted phenyl; thienyl or furanyl optionally substituted with halo;
R² is hydrogen; C₃₋₇cycloalkyl; phenyl; substituted phenyl; pyridinyl; C₁₋₆alkyl optionally monosubstituted with phenyl, C₃₋₇cycloalkyl, pyridinyl or thienyl; hydroxyl, C₁₋₆alkyloxy optionally monosubstituted with phenyl, pyridinyl, thienyl or C₃₋₆cycloalkyl; C₃₋₆alkenyloxy optionally monosubstituted with phenyl; or C₃₋₆alkynyloxy;
A is a bivalent radical having the formula
-CR³ =N- (a)
or 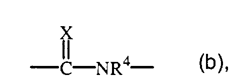 wherein the carbon atom in the bivalent radical (a) and (b) is connected to -NR²; R³ is hydrogen; C₁₋₄alkyl substituted with up to 4 halo atoms; C₃₋₇cycloalkyl; phenyl; substituted phenyl; imidazolyl; thiazolyl; thienyl; furanyl; quinolinyl; pyridinyl optionally substituted with amino, C₁₋₁₀alkyl; C₁₋₅alkyl optionally monosubstituted with phenyl, pyridinyl, imidazolyl, thienyl, indolyl or hydroxyl; C₁₋₄alkyloxy optionally monosubstituted with phenyl; C₂₋₆alkenyl optionally monosubstituted with pyridinyl, furanyl, imidazolyl or phenyl; carboxyl; C₁₋₄-alkyloxycarbonyl; phenylcarbonyl; or hydroxy and phenylmethyl;
said X being O or S;
R⁴ is hydrogen or phenylC₁₋₄alkyl;
wherein substituted phenyl is phenyl substituted with 1, 2 or 3 substituents each independently selected from halo, hydroxy, trifluoromethyl, C₁₋₆alkyl, C₁₋₆-alkyloxy, cyano, amino, mono- and di(C₁₋₆alkyl)amino, nitro, carboxyl, formyl and C₁₋₆alkyloxycarbonyl; and wherein
in the compound of formula (II) the 1H-azol-1-ylmethyl moiety is substituted on either the 5 or 6 position of the benzotriazole ring, and wherein R, R⁵, R⁶, R⁸ and-A¹=A²-A³=A⁴- in formula (II) have the following meaning
-A¹=A²-A³=A⁴- is a bivalent radical having the formula
-CH=N-CH=CH- (x);
-CH=N-CH=N- (y);
or
-CH=N-N=CH- (z);
R is hydrogen;
R⁵ is hydrogen; C₁₋₆alkyl; phenyl; substituted phenyl; C₃₋₇cycloalkyl; thienyl or furanyl optionally substituted with halo;
R⁶ is hydrogen; C₁₋₆alkyl; C₃₋₆alkenyl; C₃₋₆alkynyl; C₃₋₇cycloalkyl; phenyl; substituted phenyl; bicyclo[2.2.1]heptan-2-yl; 2,3-dihydro-1H-indenyl; 1,2,3,4-tetrahydronaphthalenyl; C₁₋₆alkyl monosubstituted with phenyl, substituted phenyl, napthalenyl, thienyl, furanyl, C₁₋₄alkylfuranyl, C₃₋₇cycloalkyl, hydroxy, C₁₋₄alkyloxy or R⁶ is a radical -OR⁷
R⁷ being hydrogen , C₁₋₆alkyl, C₃₋₆alkenyl, phenylC₃₋₆alkenyl,C₃₋₆alkynyl, pyrimidinyl, diphenylmethyl, (1-C₁₋₄alkyl-4-piperidinyl), C₁₋₆alkyl substituted with halo, hydroxy, amino, mono- or di(C₁₋₆alkyl) amino, trifluoromethyl, carboxyl, C₁₋₆alkyloxycarbonyl, phenyl, substituted phenyl, thienyl, furanyl, C₁₋₄alkylfuranyl, pyridinyl, phenoxy, phenylthio, 2,3-dihydro-1,4-benzodioxinyl, 1H-benzimidazolyl, C₁₋₄alkyl substituted 1H-benzimidazolyl, (1,1 '-biphenyl)-4-yl or 2,3-dihydro-2-oxo-1H-benzimidazolyl; and
R⁸ is hydrogen
wherein substituted phenyl is phenyl substituted with up to 3 substituents each independently selected from halo, hydroxy, hydroxymethyl, trifluoromethyl, C₁₋₆alkyl, C₁₋₆alkyloxy, C₁₋₆alkyloxycarbonyl, carboxyl, formyl, (hydroxyimino)methyl, cyano, amino, mono- and di(C₁₋₆alkyl)amino and nitro.

2. A pharmaceutical composition according to claim 1 which comprises a pharmaceutically acceptable carrier, a therapeutically effective amount of a compound of formula (I), a pharmaceutically acceptable acid addition salt or a stereochemically isomeric form thereof and a therapeutically effective amount of a retinoic acid, a derivative thereof or a stereochemically isomeric form thereof.

3. A composition according to claim 2 wherein R¹ is C₁₋₄ alkyl, phenyl, phenyl substituted with one or two halo; C₁₋₄alkyl or C₁₋₄alkyloxy substituents, or thienyl; and R² is hydrogen or C₁₋₄alkyl; R³ is hydrogen or C₁₋₄alkyl.

4. A composition according to any of claims 1 to 3 wherein the retinoic acid is *trans*-retinoic acid or 13-cis-retinoic acid.

5. A process of preparing a composition as claimed in any of claims 1 to 4, **characterized in that** the active ingredients are intimately mixed with the carrier.

6. A pharmaceutical composition according to claim 1 which comprises a pharmaceutically acceptable carrier, a therapeutically effective amount of a compound of formula (II), a pharmaceutically acceptable acid addition salt or a stereochemically isomeric form thereof and a therapeutically effective amount of a retinoic acid, a derivative thereof or a stereochemically isomeric form thereof.

7. The use of a composition as claimed in claim 6 for the manufacture of a medicament for topically treating acne.

## Patentansprüche

1. Zur topischen Anwendung geeignete pharmazeutische oder kosmetische Zusammensetzung, enthaltend einen inerten Trägerstoff, eine wirksame Menge einer Retinsäure oder eines Derivats oder einer stereochemisch isomeren Form davon und eine wirksame Menge einer Verbindung der Formel 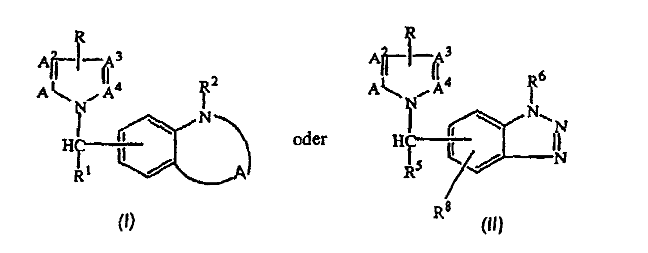 eines Säureadditionssalzes oder einer stereochemisch isomeren Form davon, wobei
die 1H-Azol-1-ylmethyl-Gruppe in der Verbindung der Formel (I) entweder an der 5- oder der 6-Stellung des Benzimidazolrings substituiert ist und wobei R, R¹, R², -A¹=A²-A³=A⁴- und A in Formel (I) die folgende Bedeutung haben
-A¹=A²-A³=A⁴- steht für einen zweiwertigen Rest der Formel
-CH=N-CH=CH- (x);
-CH=N-CH=N- (y);
oder
-CH=N-N=CH- (z);
R steht für Wasserstoff;
R¹ steht für Wasserstoff; C₁₋₆-Alkyl; C₃₋₇-Cycloalkyl; Phenyl; substituiertes Phenyl; gegebenenfalls substituiertes durch Halogen Thienyl oder Furanyl;
R² steht für Wasserstoff; C₃₋₇-Cycloalkyl; Phenyl; substituiertes Phenyl; Pyridinyl; gegebenenfalls durch Phenyl, C₃₋₇-Cycloalkyl, Pyridinyl oder Thienyl monosubstituiertes C₁₋₆-Alkyl; Hydroxyl, gegebenenfalls durch Phenyl, Pyridinyl, Thienyl oder C₃₋₆-Cycloalkyl monosubstituiertes C₁₋₆-Alkyloxy; gegebenenfalls durch Phenyl monosubstituiertes C₃₋₆-Alkenyloxy; oder C₃₋₆-Alkinyloxy;
A steht für einen zweiwertigen Rest der Formel
-CR³=N- (a)
oder 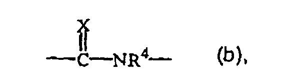 wobei das Kohlenstoffatom in dem zweiwertigen Rest (a) bzw. (b) mit -NR² verbunden ist;
R³ steht für Wasserstoff; durch bis zu 4 Halogenatome substituiertes C₁₋₄-Alkyl; C₃₋₇-Cycloalkyl; Phenyl; substituiertes Phenyl; Imidazolyl; Thiazolyl; Thienyl; Furanyl; Chinolinyl; gegebenenfalls durch Amino, C₁₋₁₀-Alkyl substituiertes Pyridinyl; gegebenenfalls durch Phenyl, Pyridinyl, Imidazolyl, Thienyl, Indolyl oder Hydroxyl monosubstituiertes C₁₋₅-Alkyl; gegebenenfalls durch Phenyl monosubstituiertes C₁₋₄-Alkyloxy; gegebenenfalls durch Pyridinyl, Furanyl, Imidazolyl oder Phenyl monosubstituiertes C₂₋₆-Alkenyl; Carboxyl; C₁₋₄-Alkyloxycarbonyl; Phenylcarbonyl; oder Hydroxyl und Phenylmethyl;
wobei X für O oder S steht;
R⁴ steht für Wasserstoff oder Phenyl-C₁₋₄-Alkyl;
wobei substituiertes Phenyl Phenyl bedeutet, das durch 1, 2 oder 3 Substituenten substituiert ist, die jeweils unabhängig voneinander aus Halogen, Hydroxyl, Trifluormethyl, C₁₋₆-Alkyl, C₁₋₆-Alkyloxy, Cyano, Amino, Mono- und Di(C₁₋₆-alkyl)amino, Nitro, Carboxyl, Formyl und C₁₋₆-Alkyloxycarbonyl ausgewählt sind; und wobei
die 1H-Azol-1-ylmethyl-Gruppe in der Verbindung der Formel (II) entweder in der 5- oder 6-Stellung des Benzotriazolrings substituiert ist, und wobei R, R⁵, R⁶, R⁸ und -A¹=A²-A³=A⁴- in der Formel (II) die folgende Bedeutung haben
-A¹=A²-A³=A⁴- steht für einen zweiwertigen Rest der Formel
-CH=N-CH=CH- (x);
-CH=N-CH=N- (y);
oder
-CH=N-N=CH- (z);
R steht für Wasserstoff;
R⁵ steht für Wasserstoff; C₁₋₆-Alkyl; Phenyl; substituiertes Phenyl; C₃₋₇-Cycloalkyl, gegebenenfalls substituiertes durch Halogen, Thienyl oder Furanyl
R⁶ steht für Wasserstoff; C₁₋₆-Alkyl; C₃₋₆-Alkenyl; C₃₋₆-Alkinyl; C₃₋₇-Cycloalkyl; Phenyl; substituiertes Phenyl; Bicyclo[2.2.1]heptan-2-yl; 2,3-Dihydro-1H-indenyl; 1,2,3,4-Tetrahydro-Naphthalinyl; durch Phenyl, substituiertes Phenyl, Naphthalinyl, Thienyl, Furanyl, C₁₋₄-Alkylfuranyl, C₃₋₇-Cycloalkyl, Hydroxyl, C₁₋₄-Alkyloxy monosubstituiertes C₁₋₆-Alkyl, oder R⁶ steht für einen Rest -OR⁷
wobei R⁷ für Wasserstoff, C₁₋₆-Alkyl, C₃₋₆-Alkenyl, Phenyl-C₃₋₆-alkenyl, C₃₋₆-Alkinyl, Pyrimidinyl, Diphenylmethyl, (1-C₁₋₄-Alkyl-4-piperidinyl), durch Halogen, Hydroxyl, Amino, Mono- oder Di(C₁₋₆-alkyl)amino, Trifluormethyl, Carboxyl, C₁₋₆-Alkyloxycarbonyl, Phenyl, substituiertes Phenyl, Thienyl, Furanyl, C₁₋₄-Alkylfuranyl, Pyridinyl, Phenoxy, Phenylthio, 2,3-Dihydro-1,4-benzodioxinyl, 1H-Benzimidazolyl, C₁₋₄-Alkyl-substituiertes 1H-Benzimidazolyl, (1,1'-Biphenyl)-4-yl oder 2,3-Dihydro-2-oxo-1H-benzimidazolyl substituiertes C₁₋₆-Alkyl steht; und
R⁸ steht für Wasserstoff,
wobei substituiertes Phenyl Phenyl bedeutet, das durch bis zu 3 Substituenten substituiert ist, die jeweils unabhängig voneinander aus Halogen, Hydroxyl, Hydroxymethyl, Trifluormethyl, C₁₋₆-Alkyl, C₁₋₆-Alkyloxy, C₁₋₆-Alkyloxycarbonyl, Carboxyl, Formyl, (Hydroxyimino)methyl, Cyano, Amino, Mono- und Di(C₁₋₆-alkyl)amino und Nitro ausgewählt sind.

2. Pharmazeutische Zusammensetzung nach Anspruch 1, enthaltend einen pharmazeutisch unbedenklichen Trägerstoff, eine therapeutisch wirksame Menge einer Verbindung der Formel (I), ein pharmazeutisch unbedenkliches Säureadditionssalz oder eine stereochemisch isomere Form davon und eine therapeutisch wirksame Menge einer Retinsäure, eines Derivats oder einer stereochemisch isomeren Form davon.

3. Zusammensetzung nach Anspruch 2, wobei R¹ für C₁₋₄-Alkyl, Phenyl, durch ein oder zwei Halogene substituiertes Phenyl; C₁₋₄-Alkyl- oder C₁₋₄-Alkyloxysubstituenten oder Thienyl steht; und R² für Wasserstoff oder C₁₋₄-Alkyl und R³ für Wasserstoff oder C₁₋₄-Alkyl steht.

4. Zusammensetzung nach einem der Ansprüche 1 bis 3, wobei es sich bei der Retinsäure um *trans*-Retinsäure oder 13-cis-Retinsäure handelt.

5. Verfahren zur Darstellung einer Verbindung nach einem der Ansprüche 1 bis 4, **dadurch gekennzeichnet, daß** man die Wirkstoffe innig mit dem Trägerstoff mischt.

6. Pharmazeutische Zusammensetzung nach Anspruch 1, enthaltend einen pharmazeutisch unbedenklichen Trägerstoff, eine therapeutisch wirksame Menge einer Verbindung der Formel (II), ein pharmazeutisch unbedenkliches Säureadditionssalz oder eine stereochemisch isomere Form davon und eine therapeutisch wirksame Menge einer Retinsäure, eines Derivates oder einer stereochemisch isomeren Form davon.

7. Verwendung einer Zusammensetzung nach Anspruch 6 zur Herstellung eines Medikaments zur topischen Behandlung von Akne.

## Revendications

1. Composition pharmaceutique ou cosmétique convenable pour l'application par voie topique, comprenant un support inerte, une quantité efficace d'un acide rétinoïque ou d'un dérivé de celui-ci, ou d'une forme stéréochimiquement isomère de celui-ci et une quantité efficace d'un composé de formule 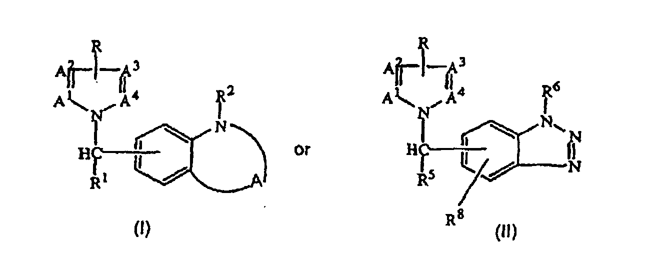 un sel d'addition d'acide ou une forme stéréochimiquement isomère de celui-ci, où
dans le composé de formule (I) le motif 1H-azol-1-ylméthyle est substitué en position 5 ou 6 du cycle benzimidazole et où R, R¹, R², -A¹=A²-A³=A⁴- et A dans la formule (I) ayant la signification suivante
-A¹=A²-A³=A⁴- est un radical bivalent ayant la formule
-CH=N-CH=CH- (x) ;
-CH=N-CH=N- (y) ;
ou
-CH=N-N=CH- (z) ;
R est hydrogène ;
R¹ est hydrogène, alkyle en C₁₋₆ ; cycloalkyle en C₃₋₇ ; phényle ; phényle substitué ; thiényle ou furanyle éventuellement substitué par halogéno ;
R² est hydrogène ; cycloalkyle en C₃₋₇ ; phényle ; phényle substitué ; pyridinyle ; alkyle en C₁₋₆ éventuellement monosubstitué par phényle, cycloalkyle en C₃₋₇, pyridinyle ou thiényle ; hydroxy, alkyloxy en C₁₋₆ éventuellement monosubstitué par phényle, pyridinyle, thiényle ou cycloalkyle en C₃₋₆ ; alkényloxy en C₃₋₆ éventuellement monosubstitué par phényle ; ou alkylnyloxy en C₃₋₆ ;
A est un radical bivalent ayant la formule
-CR³=N- (a)
ou 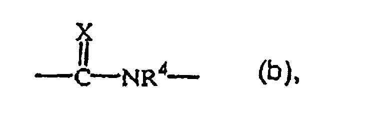 où l'atome de carbone dans le radical bivalent (a) et (b) est relié au -NR² ;
R³ est hydrogène ; alkyle en C₁₋₄ substitué par jusqu'à 4 atomes halogéno ; cycloalkyle en C₃₋₇ ; phényle ; phényle substitué ; imidazolyle ; thiazolyle ; thiényle ; furanyle ; quinolinyle ; pyridinyle éventuellement substitué par amino, alkyle en C₁₋₁₀ ; alkyle en C₁₋₅ éventuellement monosubstitué par phényle, pyridinyle, imidazolyle, thiényle, indolyle ou hydroxy ; alkyloxy en C₁₋₄ éventuellement monosubstitué par phényle ; alkényle en C₂₋₆ éventuellement monosubstitué par pyridinyle, furanyle, imidazolyle ou phényle ; carboxy ; alkyloxycarbonyle en C₁₋₄ ; phénylcarbonyle ; ou hydroxy et phénylméthyle ;
ledit X étant O ou S ;
R⁴ est hydrogène ou phénylalkyle en C₁₋₄ ;
où le phényle substitué est un phényle substitué par 1, 2 ou 3 substituants choisis chacun indépendamment parmi halogéno, hydroxy, trifluorométhyle, alkyle en C₁₋₆, alkyloxy en C₁₋₆, cyano, amino, mono- ou dialkylamino en C₁₋₆, nitro, carboxy, formyle et alkyloxycarbonyle en C₁₋₆ ; et où
dans le composé de formule (II), le motif 1H-azol-1-ylméthyle est substitué en position 5 ou 6 du cycle benzotriazole, et où R, R⁵, R⁶, R⁸ et -A¹=A²-A³=A⁴- dans la formule (II) ont la signification suivante
-A¹=A²-A³=A⁴- est un radical bivalent ayant la formule
-CH=N-CH=CH- (x) ;
-CH=N-CH=N- (y) ;
ou
-CH=N-N=CH- (z) ;
R est hydrogène ;
R⁵ est hydrogène ; alkyle en C₁₋₆ ; phényle ; phényle substitué ; cycloalkyle en C₃₋₇ ; thiényle ou furanyle éventuellement substitué par halogéno ;
R⁶ est hydrogène ; alkyle en C₁₋₆ ; alkényle en C₃₋₆ ; alkynyle en C₃₋₆ ; cycloalkyle en C₃₋₇ ; phényle ; phényle substitué ; bicyclo[2,2,1]heptan-2-yle; 2,3-dihydro-1H-indényle ; 1,2,3,4-tétrahydronaphtalényle ; alkyle en C₁₋₆ monosubstitué par phényle, phényle substitué, naphtalényle, thiényle, furanyle, alkylfuranyle en C₁₋₄, cycloalkyle en C₃₋₇, hydroxy, alkyloxy en C₁₋₄ ou R⁶ est un radical -OR⁷
R⁷ étant hydrogène, alkyle en C₁₋₆, alkényle en C₃₋₆, phénylalkényle en C₃₋₆, alkynyle en C₃₋₆, pyrimidinyle, diphénylméthyle, (1-C₁₋₄alkyl-4-pipéridinyle), alkyle en C₁₋₆ substitué par halogéno, hydroxy, amino, mono- ou dialkylamino en C₁₋₆, trifluorométhyle, carboxy, alkyloxycarbonyle en C₁₋₆, phényle, phényle substitué, thiényle, furanyle, alkylfuranyle en C₁₋₄, pyridinyle, phénoxy, phénylthio, 2,3-dihydro-1,4-benzodioxinyle, 1H-benzimidazolyle, alkyle en C₁₋₄ substitué 1H-benzimidazolyle, (1,1'-biphényl)-4-yle ou 2,3-dihydro-2-oxo-1H-benzimidazolyle ; et
R⁸ est hydrogène
où phényle substitué est un phényle substitué avec jusqu'à 3 substituants choisis chacun indépendamment parmi halogéno, hydroxy, hydroxyméthyle, trifluorométhyle, alkyle en C₁₋₆, alkyloxy en C₁₋₆, alkyloxycarbonyle en C₁₋₆, carboxy, formyle, (hydroxyimino)méthyle, cyano, amino, mono- et dialkylamino en C₁₋₆ et nitro.

2. Composition pharmaceutique selon la revendication 1 **caractérisée en ce qu'**elle comprend un support pharmaceutiquement acceptable, une quantité thérapeutiquement efficace d'un composé de formule (I), un sel d'addition d'acide pharmaceutiquement acceptable ou une forme stéréochimiquement isomère de celui-ci et une quantité thérapeutiquement efficace d'un acide rétinoïque, un dérivé de celui-ci ou une forme stéréochimiquement isomère de celui-ci.

3. Composition selon la revendication 2, **caractérisée en ce que** R¹ est alkyle en C₁₋₄, phényle, phényle substitué par un ou deux halogéno ; des substituants alkyle en C₁₋₄ ou alkyloxy en C₁₋₄, ou thiényle ; et R² est hydrogène ou alkyle en C₁₋₄ ; R³ est hydrogène ou alkyle en C₁₋₄.

4. Composition selon l'une quelconque des revendications 1 à 3, **caractérisée en ce que** l'acide rétinoïque est l'acide *trans*-rétinoïque ou l'acide 13-cis-rétinoïque.

5. Procédé de préparation d'une composition selon l'une quelconque des revendications 1 à 4, **caractérisé en ce que** les principes actifs sont mélangés intimement avec le support.

6. Composition pharmaceutique selon la revendication 1, **caractérisée en ce qu'**elle comprend un support pharmaceutiquement acceptable, une quantité thérapeutiquement efficace d'un composé de formule (II), un sel d'addition d'acide pharmaceutiquement acceptable ou une forme stéréochimiquement isomère de celui-ci et une quantité thérapeutiquement efficace d'un acide rétinoïque, un dérivé de celui-ci ou une forme stéréochimiquement isomère de celui-ci.

7. Utilisation d'une composition selon la revendication 6 pour la fabrication d'un médicament pour le traitement de l'acné par voie topique.
